(19) Europäisches Patentamt
European Patent Office
Office européen des brevets

(11) Publication number: **0 135 258**
**B1**

(12) # EUROPEAN PATENT SPECIFICATION

(45) Date of publication of patent specification: **06.09.89**

(51) Int. Cl.$^4$: **C 07 D 501/18**

(21) Application number: **84304095.7**

(22) Date of filing: **18.06.84**

(54) **Improvements in or relating to a crystalline 7-(R)-amino-3-(1-pyridiniummethyl)-ceph-3-em-4-carboxylate.**

(30) Priority: **20.06.83 US 505662**

(43) Date of publication of application:
**27.03.85 Bulletin 85/13**

(45) Publication of the grant of the patent:
**06.09.89 Bulletin 89/36**

(84) Designated Contracting States:
**BE CH DE FR GB IT LI LU NL SE**

(56) References cited:
**FR-A-2 457 295**
**US-A-4 258 041**

**Chemistry & Industry, 6(1984), 217-221**

(73) Proprietor: **ELI LILLY AND COMPANY**
**307, East McCarty Street**
**Indianapolis Indiana 46285 (US)**

(72) Inventor: **Conrad, Preston Charles**
**7632 Teel Way**
**Indianapolis Indiana 46256 (US)**
Inventor: **Jarmas, Alvydas Alfonsas**
**371 Meadowbrook Drive**
**Huntingdom Valley Pennsylvania 19006 (US)**

(74) Representative: **Crowther, Terence Roger et al**
**Erl Wood Manor**
**Windlesham Surrey GU20 6PH (GB)**

**The file contains technical information submitted after the application was filed and not included in this specification**

**EP 0 135 258 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to an intermediate in the synthesis of the cephalosporin antibiotic, 7 - (R) - [2 - (2′ - amino - 1′,3′ - thiazol - 4′ - yl) - 2 - (Z) - ((2′,2′ - dimethyl - 2′ - yl acetic acid)oxyimino ether)acetamido] - 3 - (1 - pyridiniummethyl) - ceph - 3 - em - 4 - carboxylate, also known as ceftazidime represented by Formula (1):

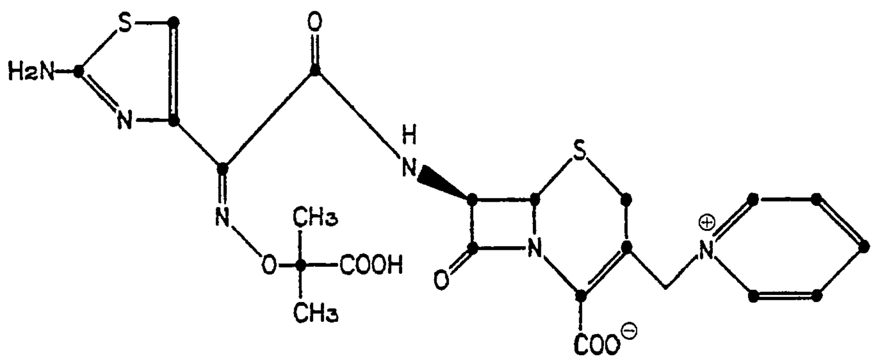

(1)

Ceftazidime is described in U.S. Patent No. 4,258,041, issued March 24, 1981. This antibiotic possesses excellent activity against a broad spectrum of both gram-positive and gram-negative pathogens.

In the production of an important antibiotic such as ceftazidime, having synthetic intermediates which are made readily in a highly purified crystalline salt form and which are stable under inexpensive storage conditions for long periods of time is advantageous.

The present invention provides a synthetic intermediate for the production of ceftazidime and which can be obtained in a stable, highly crystalline, zwitterionic form.

In accordance with the invention, there is provided 7-(R)-amino-3-(1-pyridiniummethyl)-ceph-3-em-4-carboxylate of Formula (2).

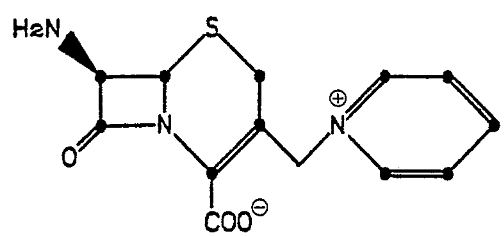

(2)

in monohydrochloride monohydrate form. As is well-known to those skilled in the art, crystalline cephalosporin derivatives tend to be more stable than the corresponding amorphous materials. Thus, in accordance with the invention, it is preferred that the compound of formula (2) be crystalline, and further that it have the following X-ray powder diffraction pattern shown in Table 1. The diffraction pattern was obtained with nickel-filtered copper radiation (Cu:Ni) of wave length $\lambda = 1.5418$Å. The interplanar spacings are in the column headed by "d" and the relative intensities in the column "$I/I_1$". (The abbreviation "b" stands for "broad").

TABLE 1

| d | $I/I_1$ |
|---|---|
| 10.75 | .47 |
| 8.17 | 1.00 |
| 6.31 | .20 |
| 5.74 | .33 |
| 5.35 | .20 b |
| 4.95 | .13 |
| 4.84 | .07 |

TABLE 1 (cont'd.)

| d | $I/I_1$ |
|---|---|
| 4.47 | .93 |
| 4.25 | .53 |
| 4.08 | 1.00 |
| 3.81 | .20 |
| 3.67 | .07 |
| 3.52 | .33 |
| 3.44 | .13 |
| 3.29 | .07 |
| 3.14 | .53 |
| 3.01 | .27 |
| 2.90 | .10 |
| 2.80 | .47 |
| 2.74 | .27 |
| 2.63 | .07 |
| 2.53 | .20 |
| 2.47 | .07 |
| 2.41 | .03 |
| 2.38 | .27 |
| 2.27 | .27 |
| 2.20 | .10 |
| 2.11 | .03 b |
| 2.06 | .03 b |
| 1.992 | .17 |
| 1.948 | .07 |
| 1.893 | .10 b |
| 1.837 | .13 |

The above polymorph has been isolated as a white microcrystalline solid.

Further, there is provided a process for preparing the monohydrochloride monohydrate which comprises dissolving the dihydrochloride dihydrate of the compound represented by Formula (2) (the "dihydrochloride dihydrate") in an aqueous solvent system and then adding dimethylacetamide. Acceptable solvent systems may include methanol/water, ethanol/water and acetone/water systems, with a 1:1 v:v methanol/water solvent system being preferred. The precipitation procedure may be carried out at a temperature of between about 25°C to about 40°C. By this procedure the monohydrochloride monohydrate is produced in crystalline form and the crystals are isolated by filtration and are dried *in vacuo* at a temperature of between about 25°C to about 40°C.

The starting material for the synthesis of the instant monohydrochloride monohydrate, *i.e.*, the corresponding dihydrochloride dihydrate, is made by cleaving the side chain (N-deacylation) of 7-(R)-(2-(thien-2-yl)acetamido)-3-(1-pyridiniummethyl)ceph-3-em-4-carboxylate, also known as "cephaloridine". The side chain of cephaloridine is cleaved by first silylating the compound, treating the silylated derivative with phosphorous pentachloride, and finally by adding isopropanol or butanediol. The precipitate of the 7-(R)-amino deacylated product obtained is dissolved in hydrochloric acid and precipitated by the addition of isopropanol to give the dihydrochloride dihydrate.

An experimental procedure for the synthesis of the dihydrochloride dihydrate is given in Preparation 10 (column 17) of O'Callaghan *et al.*, U.S. Patent No. 4,258,041, issued March 24, 1981, which is incorporated herein by reference.

The monohydrochloride monohydrate of the present invention is useful as an intermediate in the synthesis of the broad spectrum antibiotic of Formula (1), ceftazidime. More specifically, the compound represented by Formula (3):

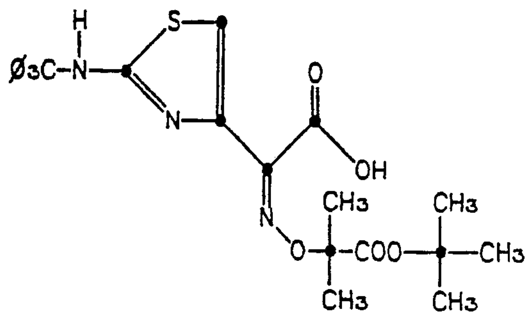

(3) ,

(2-(2'-tritylamino)-1',3'-thiazol-4'-yl)-2-(Z)-((t-butyl 2',2'-dimethyl-2'-yl acetate)oxyimino ether) acetic acid), is converted to the corresponding acid chloride with phosphorus pentachloride. The monohydrochloride monohydrate of the present invention then is acylated with the acid chloride to give 7 - (R) - [2 - (2' - (tritylamino) - 1',3' - thiazol - 4' - yl) - 2 - (Z) - ((t - butyl 2',2' - dimethyl - 2' - yl acetate) oxyimino ether)acetamido] - 3 - (1 - pyridiniummethyl) - ceph - 3 - em - 4 - carboxylate, referred to herein as "blocked ceftazidime". The amino- and carboxy-protecting groups are removed from the blocked ceftazidime by sequential treatment with concentrated formic acid and concentrated hydrochloric acid to give ceftazidime of Formula (1) as the dihydrochloride.

The conversion of the compound of Formula (3) to blocked ceftazidime is discussed below. The conversion of blocked ceftazidime to the dihydrochloride form of ceftazidime is discussed in O'Callaghan *et al.*, U.S. Patent No. 4,258,041, columns 23 and 24.

In the examples, the nuclear magnetic resonance spectra were recorded using a 90 MHz Varian Associates EM—390 instrument.

The abbreviations "mmol", "v:v", and "NMR" stand for millimole, volume to volume, and nuclear magnetic resonance spectrum, respectively. With respect to the nuclear magnetic resonance spectra, the abbreviations "d", "br s", "q", and "DMSO-$d_6$" stand for doublet, broad singlet, quartet, and dimethylsulfoxide in which all the hydrogen atoms have been replaced with deuterium, respectively.

The following non-limiting examples are provided to further illustrate the invention.

## Example 1

7-(R)-Amino-3-(1-pyridiniummethyl)ceph-3-em-4-carboxylate monohydrochloride monohydrate

7-(R)-Amino-3-(1'-pyridiniummethyl)-ceph-3-em-4-carboxylate dihydrochloride dihydrate (12.5 g, 31.25 mmol) was dissolved in 50% (v:v) methanol/water at ambient temperature. Dimethylacetamide (50 ml) was slowly added to the resultant solution. Crystallization of the monohydrochloride monohydrate began soon after the addition of dimethylacetamide was begun. Upon completion of the addition of the dimethylacetamide, the reaction mixture was cooled to 5°C for approximately 1 hour. The reaction mixture was filtered and the resultant precipitate was washed with cold (0°C) dimethylacetamide (25 ml, 1×) and then dried *in vacuo* at 40°C for 16 hours yielding 7.94 g, 73.6% (23 mmol) of 7-(R)-amino-3-(1'-pyridiniummethyl)-ceph-3-em-4-carboxylate: NMR (DMSO-$d_6$): δ 9.3 (d, J=6Hz, pyridinium protons), 8.7 (dd, 1, J=6, 6Hz, pyridinium protons), 8.2 (dd, 2, J=6, 6Hz, pyridinium protons), 5.7 (br s, 2, C—6 and C—7 protons), 5.1 (ABq, J=5Hz, 3'-methylene protons), 3.5 (ABq, 2, J=18Hz, C—2 methylene protons).

## Example 2

7-(R)-[2-(2'-(Tritylamino)-1',3'-thiazol-4'-yl)-2-(Z)-((t-butyl-2',2'-dimethyl-2'-yl-acetate)oxyimino ether)acetamido]-3-(1-pyridiniummethyl)-ceph-3-em-4-carboxylate

Phosphorus pentachloride (0.6 g, 2.98 mmol) was added to dry methylene chloride (10 ml) and the resultant solution was cooled to 0°C. 2-(2'-Tritylamino)-1',3'-thiazol-4'-yl)-2-(Z)-((t-butyl-2',2'-dimethyl-2'-yl-acetate)oxime ether)acetic acid (1.55 g, 2.71 mmol) was added to the solution and the solution was

stirred for 30 minutes at 0°C. To this solution was added a cold solution of water (7.5 ml) and triethylamine (0.9 ml), and the resultant heterogeneous mixture was stirred for 3 minutes at 0°C. The aqueous phase was separated and discarded. The remaining cold (0°C) methylene chloride phase was then added to a cooled (0°C) slurry of 7-(R)-amino-3-(1-pyridiniummethyl)-ceph-3-em-4-carboxylate monohydrochloride monohydrate (0.78 g. 2.25 mmol), dimethylacetamide (8 ml) and triethylamine (1.73 ml, 12.43 mmol) over a seven-minute period. The reaction mixture was stirred at 0°C for 60 minutes. The reaction was quenched by the addition of cold (0°C) water (18 ml), which dissolved the precipitate. The aqueous phase is separated from the organic phase and then extracted with methylene chloride (5 ml, 1×). To the combined methylene chloride extracts was added dimethylacetamide (8 ml) and diethyl ether (18 ml). The solution was heated and crystallization was allowed to take place over a thirty-minute period. The resultant thick slurry was cooled to 0°C for two hours, and the precipitate was collected by filtration, washed with a 5% dimethylacetamide/diethyl ether solution (20 ml, 1×) and then dried for 16 hours *in vacuo* at 40°C to give 1.56 g (1.85 mmol) of 7 - (R) - [2 - (2′ - (tritylamino) - 1′,3′ - thiazol - 4′ - yl) - 2 - (Z) - ((t - butyl - 2′,2′ - dimethyl - 2′ - yl - acetate)oxyimino ether)acetamido] - 3 - (1 - pyridiniummethyl) - ceph - 3 - em - 4 - carboxylate.

**Claims**

1. 7-(R)-amino-3-(1-pyridiniummethyl)-ceph-3-em-4-carboxylate monohydrochloride monohydrate.

2. A crystalline monohydrochloride monohydrate of 7-(R)-amino-3-(1′-pyridiniummethyl)-ceph-3-em-4-carboxylate having the following x-ray powder diffraction obtained with nickel-filtered copper radiation of λ = 1.5418Å wherein d represents the interplanar spacing and $I/I_1$ the relative intensity:

| d | $I/I_1$ |
|---|---|
| 10.75 | .47 |
| 8.17 | 1.00 |
| 6.31 | .20 |
| 5.74 | .33 |
| 5.35 | .20 b |
| 4.95 | .13 |
| 4.84 | .07 |
| 4.47 | .93 |
| 4.25 | .53 |
| 4.08 | 1.00 |
| 3.81 | .20 |
| 3.67 | .07 |
| 3.52 | .33 |
| 3.44 | .13 |
| 3.29 | .07 |
| 3.14 | .53 |
| 3.01 | .27 |
| 2.90 | .10 |
| 2.80 | .47 |
| 2.74 | .27 |

| d | $I/I_1$ |
|---|---|
| 2.63 | .07 |
| 2.53 | .20 |
| 2.47 | .07 |
| 2.41 | .03 |
| 2.38 | .27 |
| 2.27 | .27 |
| 2.20 | .10 |
| 2.11 | .03 b |
| 2.06 | .03 b |
| 1.992 | .17 |
| 1.948 | .07 |
| 1.893 | .10 b |
| 1.837 | .13 |

3. A process for preparing 7-(R)-amino-3-(1-pyridiniummethyl)-ceph-3-em-4-carboxylate monohydrochloride monohydrate which comprises dissolving 7-(R)-amino-3-(1-pyridiniummethyl)-ceph-3-em-4-carboxylate dihydrochloride dihydrate in an aqueous solvent system and then adding dimethylacetamide.

4. A process as claimed in claim 3 in which the solvent system is 50% methanol/water.

5. A process as claimed in claims 3 or 4 in which the crystallization is performed at about 25°C to about 40°C.

6. 7-(R)-Amino-3-(1-pyridiniummethyl)-ceph-3-em-4-carboxylate monohydrochloride monohydrate for use as an intermediate in the preparation of ceftazidime.

**Patentansprüche**

1. 7-(R)-Amino-3-(1-pyridiniummethyl)-ceph-3-em-4-carboxylatmonohydrochloridmonohydrat.

2. Kristallines Monohydrochloridmonohydrat von 7-(R)-Amino-3-(1'-pyridiniummethyl)-ceph-3-em-4-carboxylat, dadurch gekennzeichnet, daß es in einer nickelgefilterten Kupferstrahlung mit einer Wellenlänge $\lambda = 1{,}5418$Å in Pulverform das folgende Röntgenbeugungsspektrum aufweist, wobei d den Interplanarabstand bedeutet und $I/I_1$ für die relative Intensität steht:

| d | $I/I_1$ |
|---|---|
| 10,75 | 0,47 |
| 8,17 | 1,00 |
| 6,31 | 0,20 |
| 5,74 | 0,33 |
| 5,35 | 0,20 b |
| 4,95 | 0,13 |
| 4,84 | 0,07 |
| 4,47 | 0,93 |
| 4,25 | 0,53 |

| d | $I/I_1$ |
|---|---|
| 4,08 | 1,00 |
| 3,81 | 0,20 |
| 3,67 | 0,07 |
| 3,52 | 0,33 |
| 3,44 | 0,13 |
| 3,29 | 0,07 |
| 3,14 | 0,53 |
| 3,01 | 0,27 |
| 2,90 | 0,10 |
| 2,80 | 0,47 |
| 2,74 | 0,27 |
| 2,63 | 0,07 |
| 2,53 | 0,20 |
| 2,47 | 0,07 |
| 2,41 | 0,03 |
| 2,38 | 0,27 |
| 2,27 | 0,27 |
| 2,20 | 0,10 |
| 2,11 | 0,03 b |
| 2,06 | 0,03 b |
| 1,992 | 0,17 |
| 1,948 | 0,07 |
| 1,893 | 0,10 b |
| 1,837 | 0,13 |

3. Verfahren zur Herstellung von 7-(R)-Amino-3-(1-pyridiniummethyl)-ceph-3-em-4-carboxylatmonohydrochloridmonohydrat, dadurch gekennzeichnet, daß 7-(R)-Amino-3-(1-pyridiniummethyl)-ceph-3-em-4-carboxylatdihydrochloriddihydrat in einem wäßrigen Lösungsmittelsystem gelöst und die Lösung dann mit Dimethylacetamid versetzt wird.

4. Verfahren nach Anspruch 3, dadurch gekennzeichnet, daß das Lösungsmittelsystem aus 50% Methanol/Wasser besteht.

5. Verfahren nach Anspruch 3 oder 4, dadurch gekennzeichnet, daß die Kristallisation bei einer Temperatur von etwa 25 bis etwa 40°C durchgeführt wird.

6. 7-(R)-Amino-3-(1-pyridiniummethyl)-ceph-3-em-4-carboxylatmonohydrochloridmonohydrat zur Verwendung als Zwischenprodukt für die Herstellung von Ceftazidim.

**Revendications**

1. Céph-3-em-4-carboxylate de 7-(R)-amino-3-(1-pyridiniumméthyle), monochlorhydrate à une molécule d'eau.

2. Monochlorhydrate cristallin à une molécule d'eau du céph-3-em-4-carboxylate de 7-(R)-amino-3-(1'-pyridiniumméthyle), ayant le diagramme de diffraction de poudre de rayon X ci-après obtenu par rayonnement de cuivre filtre au nickel de λ = 1,5418 angström, dans lequel d représente l'écartement des plans et $I/I_1$ l'intensité relative:

| d | $I/I_1$ |
|---|---|
| 10,75 | 0,47 |
| 8,17 | 1,00 |
| 6,31 | 0,20 |
| 5,74 | 0,33 |
| 5,35 | 0,20 b |
| 4,95 | 0,13 |
| 4,84 | 0,07 |
| 4,47 | 0,93 |
| 4,25 | 0,53 |
| 4,08 | 1,00 |
| 3,81 | 0,20 |
| 3,67 | 0,07 |
| 3,52 | 0,33 |
| 3,44 | 0,13 |
| 3,29 | 0,07 |
| 3,14 | 0,53 |
| 3,01 | 0,27 |
| 2,90 | 0,10 |
| 2,80 | 0,47 |
| 2,74 | 0,27 |
| 2,63 | 0,07 |
| 2,53 | 0,20 |
| 2,47 | 0,07 |
| 2,41 | 0,03 |
| 2,38 | 0,27 |
| 2,27 | 0,27 |
| 2,20 | 0,10 |
| 2,11 | 0,03 b |
| 2,06 | 0,03 b |

| d | I/I$_1$ |
|---|---|
| 1,992 | 0,17 |
| 1,948 | 0,07 |
| 1,893 | 0,10 b |
| 1,837 | 0,13 |

3. Procédé de préparation du céph-3-em-4-carboxylate de 7-(R)-amino-3-(1-pyridiniumméthyle), monochlorhydrate a une molécule d'eau, ce procédé comprenant les étapes consistant à dissoudre le céph-3-em-4-carboxylate de 7-(R)-amino-3-(1-pyridiniumméthyle), dichlorhydrate à deux molécules d'eau, dans un système de solvants aqueux et à ajouter ensuite du diméthylacétamide.

4. Procédé selon la revendication 3, dans lequel le systéme de solvants est un mélange de méthanol/eau à 50%.

5. Procédé selon la revendication 3 ou 4, dans lequel on réalise la cristallisation à une température comprise dans l'intervalle allant d'environ 25°C à environ 40°C.

6. Céph-3-em-4-carboxylate de 7-(R)-amino-3-(1-pyridiniumméthyle), monochlorhydrate à une molécule d'eau, pour l'utilisation comme produit intermédiaire dans la préparation de la ceftazidime.